# EUROPEAN PATENT APPLICATION

(11) **EP 4 796 524 A1**
(43) Date of publication of application: **26.08.2026**
(21) Application number: 25159710.0
(22) Date of filing: 24.02.2025
(51) Int. Cl.: C07C 37/055, C07C 39/16, C08J 11/00

(54) **BISPHENOL A RECOVERY FROM THERMOSET EPOXY RESINS USING ALCOHOLYSIS AND RELATED METHODS**

(71) Applicant: VITO NV, 2400 Mol (BE)
(72) Inventor: MARQUEZ ADMADE, Carlos, 2400 Mol (BE); FEGHALI, Elias, 2400 Mol (BE); ELST, Kathy, 2400 Mol (BE)
(74) Representative: Arnold & Siedsma

(57) **Abstract**

The method involves recovering Bisphenol A (BPA) monomer from a thermoset BPA-epoxy resin through alcoholysis using a C1-C3 alcohol and a hydroxide as a reaction promoter. This process is conducted under an inert atmosphere at a pressure of at least 10 bar and a temperature between 200-225°C for a minimum of 5 hours. The method allows for high yields of BPA monomer with reduced reaction times and less base usage compared to existing methods. The process includes loading 2-10 wt.% of the resin in the alcohol and can involve further steps such as crystallization and recrystallization for purification.

## Description

### TECHNICAL FIELD

The present disclosure relates to a method for recovering Bisphenol A (BPA) from BPA-based thermoset epoxy resins and fiber reinforced composites using alcoholysis with lower alkyl alcohols in the presence of sodium hydroxide.

### BACKGROUND

Epoxy resins are a class of reactive prepolymers and polymers which contain epoxide groups. They are widely used in a variety of industrial applications due to their strong adhesive properties, chemical resistance, and durability. One of the primary components in the production of epoxy resins is bisphenol A (BPA), a chemical compound that has been under scrutiny due to its potential health and environmental impacts. BPA is used extensively in the manufacture of polycarbonate (PC) plastics and epoxy resins, which are found in numerous consumer goods, including water bottles, food containers, and coatings for metal products such as food cans, bottle tops, and water supply pipes.

Thermoset epoxy resins are a type of polymer prepared by blending a component bearing an epoxy function (usually bisphenol A-based, BPA) with a curing agent (amines or anhydrides).¹ Unlike thermoplastics (like PC), thermosets are not considered recyclable, due to the (strong) covalent bonds formed during the curing process. These thermosets are widely used in construction, electronic and manufacturing applications as coating and adhesives for structural and other purposes. However, their major application is undoubtedly in fiber-reinforced epoxy composites.² Epoxy resins comprise ~50 wt.% of carbon fiber or ~40 wt.% of glass fiber composite materials. ²⁻⁴ Carbon fiber reinforced polymer (CFRPs) composites have been extensively used in the aerospace, transport, marine and wind energy sector for more than five decades. The annual growth rate of CFRPs is about 12.5% over the last two decades, with a global demand of 4 million metric tons in 2020.^{5,6} On the other hand, glass fiber reinforced polymer (GFRPs) composites find applications in diverse engineering fields and are the preferred material for (wind turbine) blade skin manufacturing. Nevertheless, recycling solutions for end-of-life (EOL) composite materials are critically underdeveloped, while the amount of EOL GFRP wind turbine blades alone expected to reach around 30 kT per year between 2020 and 2030. ^{4,7,8}

Most common disposal methods for composite waste involve landfilling, pyrolysis, or mechanical recycling by grinding composites for use in applications that can tolerate lower-quality mechanical properties.⁹⁻¹² Even though emerging chemical recycling strategies primarily focus on recycling of the fiber, the epoxy portion of these composites also represents a substantial amount of unrecovered carbon.^{2,3} Together with an increasing concern over BPA's environmental and health effects, this has led to a growing demand for effective methods to recover and recycle BPA from thermoset epoxy resins.

Traditional methods of BPA recovery often involve complex processes that can be time-consuming and require significant amounts of chemical reagents, which may not be environmentally friendly. Up until now only a few reports tackle the challenging chemical recycling of amine-cured epoxies back to bisphenol A (BPA), with breakthrough methods based on homogeneous Ru and Ni-based catalysts, metal alkoxide and hydroxide bases (Table 1). However, these methods depend on exceedingly high amounts of catalysts (which are expensive and sensitive to oxygen, water and impurities) or base, or very long reaction times to obtain decent yields of BPA.

For example, in references 2 and 7 the authors use non-reactive apolar solvents requiring high amounts of base (4 eq. = ~50 wt.%) and very long reactions times (1 to 2 days). In references 14 and 15, the authors perform a transfer hydrogenation (H₂ as reagent generated in situ to break the epoxy bonds) and use a homogeneous (and very expensive, non-abundant metal) catalyst and very long reaction times (4 days). In reference 16, the authors perform hydrolysis (with supercritical water as reagent to break the epoxy bonds). They do not report the amount of catalyst used but they only recover BPA in very low yields. In reference 17, the authors perform a digestion/deconstruction of the epoxy composite. It is solvent free but with exceedingly high amounts of a molten NaOH-KOH eutectic mixture (up to 10000 wt.% compared to the composite).

**Table 1. Summary of chemical recycling strategies for amine-cured epoxy resins for BPA recovery**

| **Solvent** | **Catalyst/Promoter** | **Temperature (°C)** | **Time (h)** | **BPA yield (%)** | **Reference** |
|---|---|---|---|---|---|
| THF/toluene | KOtBu (4 eq.) | 160 | 48 | 71 | 2 |
| Toluene | Triphos-Ru-TMM (3 mol%) | 160 | 96 | 81 | 14 |
| Toluene | NaOH (50 wt.%) | 190 | 24 | 81 | 7 |
| N-Methyl-2-pyrrolidone | Ni(cod)(dcype) (10 mol%) | 200 | 36 | 66 | 15 |
| Supercritical water | NH₄Cl (amount not reported) | 260 | 1 | 25 | 16 |
| Molten NaOH-KOH eutectic mixture | NaOH-KOH (1000-10000 wt. %) | 220 | 0.5-3 | >85 | 17 |

As industries strive to adopt more sustainable practices, there is a pressing need for innovative solutions that can efficiently recover BPA while minimizing environmental impact and resource consumption. This need drives the exploration of new methodologies that can offer improved efficiency and sustainability in BPA recovery processes.

### SUMMARY

The method may involve recovering Bisphenol A (BPA) monomer from a thermoset BPA-epoxy resin. The process can include subjecting the BPA-epoxy resin to alcoholysis using a C1-C3 alcohol, with up to 50 wt.% of an alkali hydroxide as a reaction promoter. The reaction may occur under an inert atmosphere at a pressure of at least 10 bar and a temperature between 180-250°C for at most 24 hours. The alcoholysis can be performed by loading 2-10 wt% of the thermoset BPA-epoxy resin in the C1-C3 alcohol, thereby potentially recovering the BPA monomer in high yields with shorter reaction times while reducing the amount of base used compared to some existing methods.

Some examples of the method may specify that the C1-C3 alcohol is selected from ethanol, methanol, or propanol, with methanol being a particular choice.

The inert atmosphere used in the method can be argon.

Alternatively, the inert atmosphere may be nitrogen.

The pressure during the process can range from 10-50 bar, with a preference for 20 or 40 bar.

The temperature for the method may be set between 200 - 250°C; in particular from about 200°C up to about 225°C.

The alcoholysis process can be carried out for at least 5 hours, in particular for a duration of 2 to 15 hours, more in particular for a duration of 5-10 hours.

Some examples of the method may specify that the alkali hydroxide used as promoter is selected from NaOH, LiOH, or KOH, with NaOH being a particular choice.

The amount of alkali hydroxide promoter used in the method is at least 5 wt.% and may for example range from 10-40 wt.%, with a particular range of 15 to 25 wt.%.

The thermoset BPA-epoxy resin can be loaded in an amount of 3-8 wt.% in the C1-C3 alcohol.

The method may also include recovering the BPA monomer from the reaction mixture by crystallization.

Additionally, the recovered BPA monomer can be purified by recrystallization from a suitable solvent.

Some examples of suitable solvents for recrystallization may include water, methanol, ethanol, isopropanol, acetone, and ethyl acetate.

The method may achieve a conversion of at least 90%, preferably at least 95%, more preferably at least 98% of the thermoset BPA-epoxy resin subjected to alcoholysis into depolymerization products, in particular with a yield of at least 20% of BPA.

In the method wherein, the thermoset BPA-epoxy resin can be part of fiber-reinforced epoxy composites such as carbon fiber or glass fiber composite materials.

### BRIEF DESCRIPTION OF THE DRAWINGS

FIG. 1 illustrates a process for efficient depolymerization and recovery of BPA from epoxy resin.
FIG. 2 illustrates the effect of NaOH loading in BPA yield (at different reaction times). Reaction conditions: T = 200°C, P = 20 bar Ar, 5 wt% epoxy loading in MeOH, NaOH as promoter.
FIG. 3 summarizes the effect of temperature in the depolymerization (methanolysis) of amine-cured epoxy resins at different NaOH loadings. X represents epoxy resin conversion and Y_{BPA} is the BPA yield after 5 h of reaction time. Reaction conditions: P = 20 bar Ar, 5 h, 5 wt.% epoxy loading, NaOH as promoter.
FIG. 4 illustrates the effect of an inert gas (Ar) pressure in the depolymerization (methanolysis) of amine-cured epoxy resins. Reaction conditions: T = 200 °C, 5 h reaction time, 5 wt.% polymer loading (in MeOH), 20 wt.% NaOH.
FIG.5 illustrates the reaction scheme for the present process of depolymerization and recovery of BPA from epoxy resin. In said scheme ROH represents the C1-C3 alcohol, and formula I corresponds to the polyol fraction containing the curing agent. As further detailed in the examples, the obtained products were analyzed by gas chromatography (GC). Further identification of the polyol fraction containing the curing agent could for example be done using liquid chromatography-mass spectrometry (LC-MS).

### DETAILED DESCRIPTION

This disclosure provides a method for recovering Bisphenol A (BPA) monomer from thermoset BPA-epoxy resin through alcoholysis using lower alkyl alcohols such as methanol, ethanol, or propanol; in the presence of an alkali hydroxide as reaction promoter. The process may involve loading 2 to 10 wt.% of the resin in the alcohol, with the alkali hydroxide acting as a reaction promoter in amounts ranging from 15 to 50 wt.%. The reaction is conducted under an inert atmosphere, such as argon or nitrogen, at a pressure of at least 10 bar and a temperature between 180 to 250°C, with reaction times of at least 5 hours and up to at most 24 hours. This method may achieve high monomer yields in shorter reaction times while minimizing the amount of base used. The BPA monomer can be recovered from the reaction mixture by crystallization and further purified by recrystallization using suitable solvents like water, methanol, ethanol, isopropanol, acetone, or ethyl acetate. The process may convert at least 90% of the resin into depolymerization products, in particular yielding at least 20% of BPA without further purification steps.

As used herein, an alkali hydroxide is a compound that consists of an alkali metal (such as sodium, potassium, or lithium) combined with a hydroxide ion (OH⁻). These compounds are typically strong bases and are highly soluble in water. Some common examples include: Sodium hydroxide (NaOH), also known as lye or caustic soda; Potassium hydroxide (KOH), often used in soap making; and Lithium hydroxide (LiOH), used in batteries and air purification systems.

As used herein, the thermoset BPA-epoxy resin includes fiber-reinforced epoxy composites such as carbon fiber or glass fiber composite materials; in particular carbon fiber reinforced polymer (CFRPs) composites. Hence according to an embodiment of the invention it provides a method for recovering Bisphenol A (BPA) monomer from a fiber-reinforced epoxy composite, the method comprising: - subjecting the fiber-reinforced epoxy composite to alcoholysis using a C1-C3 alcohol in the presence of at least 5 wt.% an alkali hydroxide, in particular NaOH as a reaction promoter, under an inert atmosphere at a pressure of at least 10 bar and a temperature in the range of 200-225°C for at least 5 hours, wherein the alcoholysis is carried out by loading 2 -10 wt.% of the fiber-reinforced epoxy composite in the C1-C3 alcohol, thereby recovering the BPA monomer in high yields with shorter reaction times while reducing the amount of base used compared to existing methods.

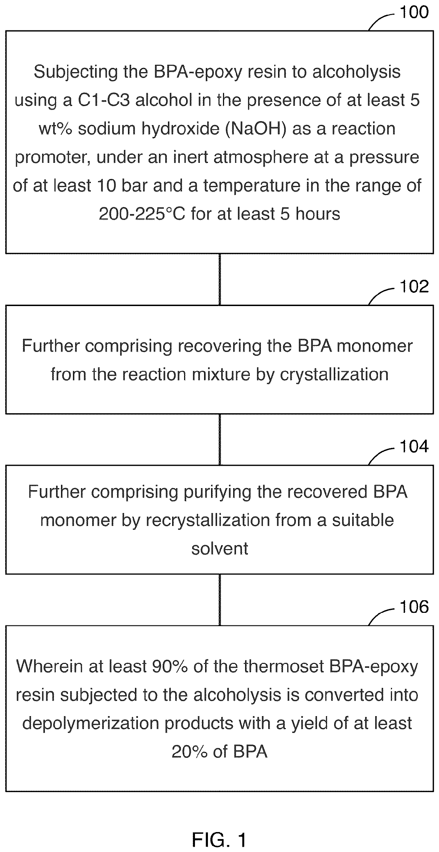

FIG. 1 is a flowchart illustrating a method in step 100 for recovering Bisphenol A (BPA) monomer from a thermoset BPA-epoxy resin via alcoholysis, according to an embodiment. At step 100, the BPA-epoxy resin may be subjected to alcoholysis using a C1-C3 alcohol, such as methanol, ethanol, or propanol, in for example the presence of sodium hydroxide (NaOH) as a reaction promoter. The process occurs under an inert atmosphere, which can be either argon or nitrogen, at a pressure of at least 10 bar and within a temperature range of 200-225°C. The reaction may be maintained for at least 5 hours to ensure effective alcoholysis. In an embodiment the method may involve loading 2-10 wt% of the thermoset BPA-epoxy resin in the C1-C3 alcohol, with the alkali hydroxide promoter, preferably NaOH potentially ranging from 10-40 wt.%, particularly between 15 to 25 wt%. The pressure may be adjusted within the range of 10-50 bar, with a preference for 20 or 40 bar, while the temperature may be fine-tuned to 210-220°C. The alcoholysis duration may extend to 5-10 hours, allowing for the conversion of at least 90% of the thermoset BPA-epoxy resin into depolymerization products, achieving a yield of at least 20% of BPA. This method may facilitate the recovery of BPA monomer in high yields with shorter reaction times, while reducing the amount of base used compared to existing methods.

In the context of recovering bisphenol A (BPA) monomer from a thermoset BPA-epoxy resin, step 102 may involve the process of recovering the BPA monomer from the reaction mixture through crystallization. This step may ensure that the BPA monomer is effectively separated from the other components present in the reaction mixture. The crystallization process may be facilitated by the conditions established in the preceding steps, where the BPA-epoxy resin is subjected to alcoholysis using a C1-C3 alcohol, such as methanol, in the presence of an alkali hydroxide such as sodium hydroxide (NaOH) in particular, as a reaction promoter. The reaction may occur under an inert atmosphere, such as argon or nitrogen, at a pressure of at least 10 bar and a temperature range of 200-225°C for a duration of at least 5 hours. These conditions may promote the breakdown of the BPA-epoxy resin, allowing the BPA monomer to be released into the reaction mixture. The crystallization step may then enable the BPA monomer to be recovered in a purified form, potentially leading to high yields and shorter reaction times compared to existing methods. This process may also reduce the amount of base used, contributing to a more efficient and sustainable recovery method. The crystallization of the BPA monomer may be a step in achieving the desired purity and yield, ensuring that the recovered monomer is suitable for further use or processing.

In step 104, the process may involve purifying the recovered Bisphenol A (BPA) monomer through recrystallization using a suitable solvent. This step may ensure the purity of the BPA monomer, which can be achieved by selecting solvents such as water, methanol, ethanol, isopropanol, acetone, or ethyl acetate. The choice of solvent may depend on the specific conditions and desired purity levels. The recrystallization process may involve dissolving the BPA monomer in the chosen solvent under controlled conditions, allowing the impurities to remain in solution while the pure BPA monomer crystallizes out. This step may enhance the quality of the recovered BPA, making it suitable for further applications or studies. The process may be carried out with precision to ensure that the maximum amount of pure BPA is obtained, thereby optimizing the recovery process. The use of different solvents may offer flexibility in the purification process, allowing for adjustments based on the specific requirements of the BPA recovery method.

In the context of Step 106, the process may involve the conversion of at least 90% of the thermoset BPA-epoxy resin subjected to alcoholysis into depolymerization products, with a yield of at least 20% of Bisphenol A (BPA). This step may be part of the overall method for recovering BPA monomer from BPA-based epoxy resins. The conversion process may be facilitated by the use of lower alkyl alcohols, such as methanol, ethanol, or propanol, which may act as solvents in the alcoholysis reaction. With reference to Table 4 below, the presence of an alkali hydroxide as a reaction promoter provides quasi full conversion yields (at least 90%), potentially leading to higher yields of BPA. The reaction may be conducted under an inert atmosphere, such as argon or nitrogen, to prevent unwanted side reactions and to maintain the integrity of the reaction environment. The pressure and temperature conditions, which may be maintained at a pressure of at least 10 bar and within a temperature range of 200 to 225°C, respectively, may be optimized to ensure the effective depolymerization of the resin. The process may also benefit from incubation times of at least 5 hours, allowing sufficient time for the reaction to proceed to completion. The conversion of the resin into depolymerization products may be a step in achieving the desired yield of BPA, which may be further recovered and purified in subsequent steps. This method may offer advantages over existing methods by potentially reducing the amount of base used and achieving high monomer yields in shorter reaction times.

### EXAMPLES

With specific reference to the figures and examples hereinafter, it is emphasized that the particulars shown are only for example purposes and only for the illustrative discussion of the various embodiments of the present invention. They are presented with the aim of providing what is considered to be the most useful and effective description of the principles and conceptual aspects of the invention. In this respect, no attempt is made to show more structural details of the invention than is necessary for a fundamental understanding of the invention. The description in combination with the figures and examples makes it clear to the experts in the field how the different forms of the invention can be implemented in practice.

### Materials and methods

All commercially available solvents, reagents, and chemicals were used as received without further purification unless otherwise stated. Tetrabutylammonium bromide (TBAB), epichlorohydrin (ECH), NaOH, KOH, Ba(OH)₂, Sr(OH)₂, KOtBu, Ca(OH)₂, Na₂CO₃, Na₂SO₄, MeOH, EtOH, 2-propanol, toluene, DCM and ethylene glycol were purchased from VWR Chemicals. Bisphenol A (BPA) and JEFFAMINE D-230 polyetheramine (poly(propylene glycol) bis(2-aminopropyl ether) Mn=230 g/mol) were purchased from Sigma Aldrich.

### Synthesis of bisphenol A diglycidylether (BADGE):

In a 250 mL round bottom flask 27 g of BPA were mixed with 0.88 g of TBAB and 15 g of KOH in 50 mL of ECH used in excess as reactant and solvent. The reaction mixture was stirred at room temperature for 16 h treated with water and extracted three times with DCM. The recovered organic fractions were washed with distilled water and dried by anhydrous Na₂SO₄. The DCM was removed, and the crude purified via silica gel chromatography using hexane 4:1 ethyl acetate as eluent. The obtained BADGE was used in the following step without further purification.

### Thermoset (epoxy resins) synthesis:

The epoxy resins used in the depolymerization experiments were prepared by modification of a procedure reported in ref. 18. In short, the thermoset was prepared by mixing equimolar amounts of (BADGE) and JEFFAMINE D-230 polyetheramine in a 20 ml vial under vigorous stirring. The polymer thermosets were synthesized by depositing the reaction mixture on a Teflon mould and pre-cured in the hot-press at 90 °C for 15 min under 1 metric Ton allowing the mixture to increase the viscosity. Then, the moulds were hot-pressed at 130 °C for 2 h under 2 metric Ton to complete the curing process. With the mould still warm the epoxy samples were removed, cut in the desired dimensions, and allowed to cool down to room temperature. The polymer was then directly used in deconstruction reactions without additional purification.

### Thermoset (epoxy resins) depolymerization reactions:

A 100 ml high-pressure batch reactor (Premex) was used for the reductive depolymerization of epoxy resins. To this end, the reactor was filled with 2.3 g of polymer and 46 g of the solvent. Then, the required amounts of base were loaded and the reactor was sealed, pressurized with Ar, heated to the desired temperature and stirred at 750 rpm for a specific time. After completion of the reaction, the reactor was cooled down in a water bath and opened to collect the product mixture. The unreacted polymer was recovered by filtration and the conversion was calculated by comparing it to the original epoxy resin weight (2.3 g). The product mixture (filtrate) was analyzed by gas chromatography (GC) to determine the selectivity to BPA.

### Gas Chromatography:

Products were analysed by gas chromatography (GC) in a system equipped with a combi-pal series injector, DB5-MS column (60 m x 0.25 mm ID x 0.25 mm), and a flame ionization detector (FID) operating at 310 ° C. The Chromeleon software was used for the analysis. An automated injector injected 1 *µ* L of sample with a split ratio 14.3 at a split flow 10. The injection port and initial oven temperatures were 300 ° C and 40 ° C, respectively. The oven 5 temperature was increased and kept constant for 13 min at 265 ° C, for a total run time of 30 min.

### Results

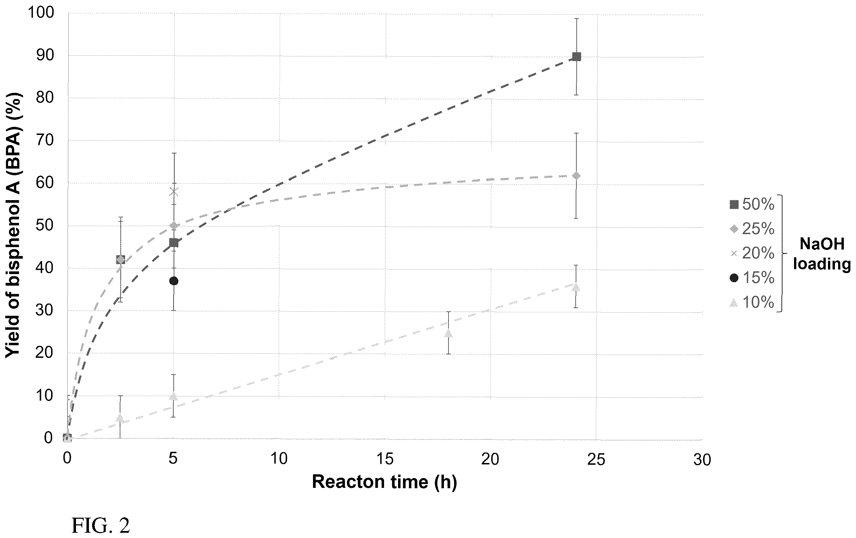

In assessing the possible depolymerization of BPA based thermoset epoxy resins using methanolysis we first tested the effect of NaOH loading in BPA yields at different reaction times using a reaction temperature of 200°C and an inert atmosphere under 20 bar Ar. The results are summarized in Table 2 below and Figure 2.

NaOH loads of at least 10 wt.% are needed to see a quasi-full (at least 95%) conversion, i.e depolymerization, of the amine-cured epoxy resins. Surprisingly, at a NaOH load of 20 wt.% the BPA yield after 5 hours is higher than the BPA yields with higher NaOH loads, 25 wt.% and 50 wt.% respectively, suggesting that at this concentration the reaction kinetics are higher, resulting in a faster depolymerization with high yields in BPA.

**Table 2. Effect of NaOH in the depolymerization of amine-cured epoxy resins (blank reaction comparison).**

| NaOH loading | Epoxy conversion (%) | BPA yield (%) |
|---|---|---|
| 15 wt.% | >99% | 37% |
| 10 wt.% | 95% | 29% |
| No NaOH | <5% | <1% |

| | | |
|---|---|---|
| Reaction conditions: T = 225 °C, P = 20 bar Ar, 5 h, 5 wt.% epoxy loading (in MeOH), NaOH as promoter. | | |

NaOH loads of at least 10 wt.% are needed to see a quasi-full (at least 95%) conversion, i.e depolymerization, of the amine-cured epoxy resins. Surprisingly, and with reference to Figure 2 at a NaOH load of 20 wt.% the BPA yield after 5 hours is higher than the BPA yields with higher NaOH loads, 25 wt.% and 50 wt.% respectively, suggesting increased reaction kinetics and increased selectivity to the desired product (BPA) over smaller phenolic molecules. Notable, in the absence of NaOH depolymerization does not occur.

As this initial experiment demonstrated the possibility of depolymerization of BPA based thermoset epoxy resins using methanolysis, we further tested the possibility of using different solvents. As shown in Table 3 below, lower alkyl alcohols could effectively be used with Epoxy conversions of at least 95% and BPA yields in the same range as with methanol.

**Table 3. Effect of solvent on the depolymerization of amine-cured epoxy resins.**

| Solvent | Epoxy conversion (%) | BPA yield (%) |
|---|---|---|
| Methanol | 96% | 57% |
| Ethanol | 97% | 49% |
| 2-Propanol | 98% | 62% |
| Toluene | <5% | <1% |
| Ethylene glyc | ol <5% | <1% |

| | | |
|---|---|---|
| Reaction conditions: T = 200 °C, P = 20 bar Ar, 5 h, 5 wt.% epoxy loading, 20 wt.% NaOH. | | |

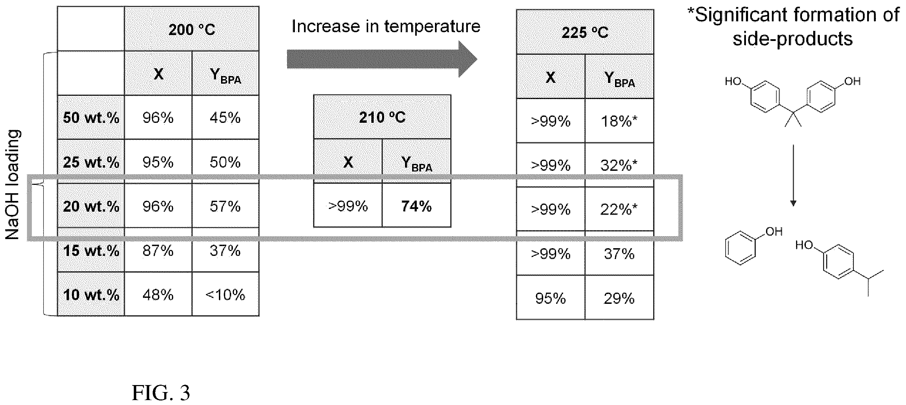

From Figure 2 and the tables above, it could already be seen that there is an effect of the reaction temperature on the BPA yields. Based on a further analysis it appears that at higher temperature the liberated BPA is further degraded with a significant formation of side products. As summarized in Figure 3, an optimum in epoxy conversion and BPA yields is realized at a temperature of 210°C.

Having established that lower alkyl alcohols could be used as solvent, we also investigated if different alkaline promoters could be used in the depolymerization the BPA-based thermoset epoxy resins. Surprisingly, and as summarized in table 4 below, the tested alkali hydroxides can fully depolymerize the epoxy resin, with NaOH showing the highest epoxy conversion of at least 99% and the highest BPA yields (74%).

**Table 4. Effect of base (promoter) in the depolymerization (methanolysis) of amine-cured epoxy resins.^{a}**

| Base | Epoxy conversion (%) | BPA yield (%) |
|---|---|---|
| NaOH | 99% | 74% |
| KOH^{b} | 98% | 10% |
| LiOH^{b} | 99% | 22% |
| KOtBu | 13% | <5% |
| Ba(OH)₂ | 57% | <10% |
| Sr(OH)₂ | 8% | <4% |
| Ca(OH)₂ | <5% | <1% |
| Na₂CO₃ | <5% | <1% |

| | | |
|---|---|---|
| ^{a} Reaction conditions: T = 210 °C, P = 20 bar Ar, 5 h reaction time, 5 wt.% polymer loading (in MeOH), 11.5 mmol of base. ^{b} Reaction conditions: T = 210 °C, P = 40 bar Ar, 24 h reaction time, 5 wt.% polymer loading (in MeOH), 23 mmol of base | | |

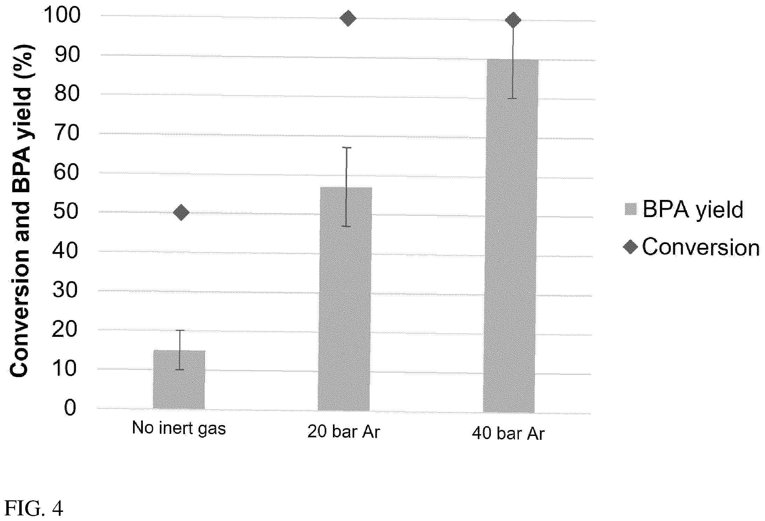

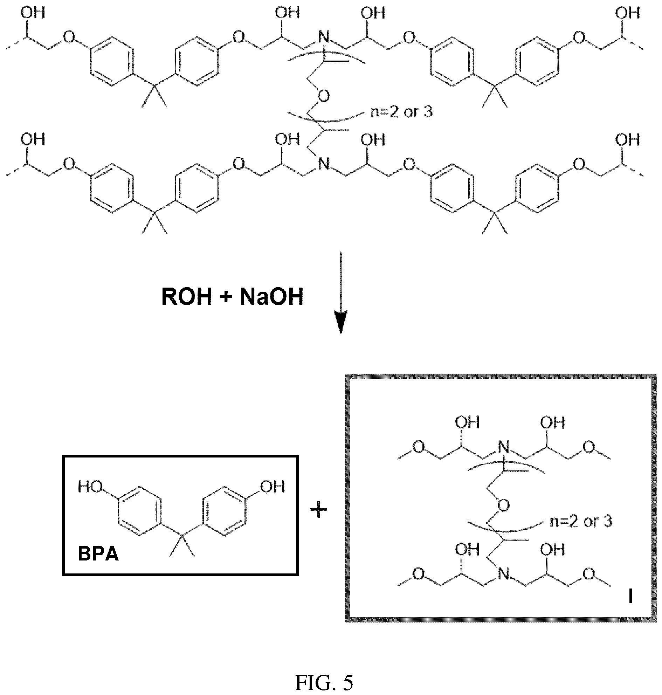

In short, and as schematically shown in the reaction scheme of Figure 5, we report a base-promoted depolymerization of amine-cured epoxy resins via alcoholysis, using a hydroxide for the selective C(alkyl)-O bonds cleavage (i.e. C(aryl)-O, C-N and C-C bonds are kept intact) for the recovery of BPA and a polyol fraction containing the curing agent (shown as formula I in Figure 5). Our results show that under our optimal conditions (Figure 4), (T = 200 °C, P = 40 bar Ar, 5 h of reaction time, 5 wt.% of polymer loading in MeOH and 20 wt.% NaOH) we achieve a full Epoxy conversion with a BPA yield of 90% (±10).

### References

1. H. Q. Pham and M. J. Marks, Ullmann's Encyclopedia of Industrial Chemistry, Wiley-VCH Verlag GmbH & Co. KGaA, 2005.
2. R. C. DiPucchio, K. R. Stevenson, C. W. Lahive, W. E. Michener and G. T. Beckham, ACS Sustainable Chem. Eng. 2023, 11, 16946-16954.
3. S. R. Nicholson, N. A. Rorrer, A. C. Carpenter and G. T. Beckham. Joule. 2021, 5, 673-686.
4. N. Papadakis, C. Ramírez and N. Reynolds, Designing Composite Wind Turbine Blades for Disposal, Recycling or Reuse. In Management, Recycling and Reuse of Waste Composites; Woodhead Publishing: Sawston, UK, 2010; pp. 443-457.
5. E. Linak, U. Buchholz, M. Guan and A. Kishi. IHS Report on Epoxy Resins, 2020*.*
6. J. Zhang, G. Lin, U. Vaidya and H. Wang. Compos. B: Eng. 2023, 250, 110463.
7. H. Sun, A. Ahrens, G. M. F. Batista, B. S. Donslund, A. K. Ravn, E. V. Schwibinger, A. Nova and T. Skrydstrup, Green Chem., 2024, 26, 815-824.
8. M. Diani and M. Colledani, Procedia CIRP, 2020, 90, 483-487.
9. S. K. Gopalraj and T. A. Kärki, SN Appl. Sci., 2020, 2, 433.
10. C. Ma, D. Sánchez-Rodríguez and T. Kamo, J. Hazard. Mater., 2021, 412, 125329.
11. S. R. Naqvi, H. M. Prabhakara, E. A. Bramer, W. Dierkes, R. Akkerman and G. Brem, Resour. Conserv. Recycl. 2018, 136, 118-129.
12. P. Liu and C. Y. Barlow, Waste Manage. 2017, 62, 229-240.
13. A. Cooperman, A. Eberle and E. Lantz, Resour. Conserv. Recycl. 2021, 168, 105439.
14. A. Ahrens, A. Bonde, H. Sun, N. K. Wittig, H. C. D. Hammershøj, G. M. F. Batista, A. Sommerfeldt, S. Frølich, H. Birkedal and T. Skrydstrup, Nature, 2023, 617, 730-737.
15. Y. Liao, K. Takahashi and Kyoko Nozaki, J. Am. Chem. Soc. 2024, 146, 2419-2425.
16. Y. J. Lim, Z. Yu, V. Cherepakhin, T. J. Williams and S. R. Nutt, Green Chem., 2025, Advance Article. DOI:10.1039/D4GC05299D.
17. L. Yuyan, L. Li, W. Songquan and L. Yuting, Polym. Polym. Compos., 2012, 20, 9, 809-816.
18. M. Comí, M. Thys, A. Aerts, S. Geudens, S. Vloemans, E. Feghali, K. Vanbroekhoven and R. Vendamme, ChemSusChem, 2025. DOI:10.1002/cssc.202402375.

## Claims

1. A method for recovering Bisphenol A (BPA) monomer from a thermoset BPA-epoxy resin, the method comprising:
- subjecting the BPA-epoxy resin to alcoholysis using a C1-C3 alcohol in the presence of at most 50 wt.% of an alkali hydroxide as a reaction promoter, under an inert atmosphere at a pressure of at least 10 bar and a temperature in the range of 180 to 250°C for at most 24 hours,
wherein the alcoholysis is carried out by loading 2 -10 wt.% of the thermoset BPA-epoxy resin in the C1-C3 alcohol,
thereby recovering the BPA monomer in high yields with shorter reaction times while reducing the amount of base used compared to existing methods.

2. The method according to claim 1, wherein the C1-C3 alcohol is selected from ethanol, methanol or propanol; in particular methanol.

3. The method according to claim 1, wherein the inert atmosphere is argon.

4. The method according to claim 1, wherein the inert atmosphere is nitrogen.

5. The method according to claim 1, wherein the pressure is in the range of 10-50 bar; preferably at 20 or 40 bar.

6. The method according to claim 1, wherein the temperature is in the range of 200-220°C.

7. The method according to claim 1, wherein the alcoholysis is carried out for 2 to 15 hours, in particular for 5-10 hours.

8. The method according to claim 1, wherein the alkali hydroxide promoter is selected from NaOH, KOH or LiOH; in particular NaOH.

9. The method according to claim 1, wherein the amount of reaction promoter is at least 5 wt%, in particular in the range of 10-50 wt%; more in particular in the range of 15 to 25 wt.%.

10. The method according to claim 1, wherein the thermoset BPA-epoxy resin is loaded in an amount of 3-8 wt.% in the C1-C3 alcohol.

11. The method according to claim 1, further comprising recovering the BPA monomer from the reaction mixture by crystallization.

12. The method according to claim 1, further comprising purifying the recovered BPA monomer by recrystallization from a suitable solvent.

13. The method according to claim 12, wherein the suitable solvent is selected from the group consisting of water, methanol, ethanol, isopropanol, acetone, and ethyl acetate.

14. The method according to claim 1, wherein at least 90% of the thermoset BPA-epoxy resin subjected to the alcoholysis is converted into depolymerization products with a yield of at least 20% of BPA.

15. The method according to claim 1, wherein the thermoset BPA-epoxy resin is part of fiber-reinforced epoxy composites such as carbon fiber or glass fiber composite materials.
